(19) Europäisches Patentamt
European Patent Office
Office européen des brevets

(11) Publication number: 0 263 208 A2

# (12) EUROPEAN PATENT APPLICATION

(21) Application number: 86307770.7

(51) Int. Cl.[4]: A61K 31/165

(22) Date of filing: 08.10.86

(43) Date of publication of application: 13.04.88 Bulletin 88/15

(84) Designated Contracting States: AT ES GR

(71) Applicant: THE UPJOHN COMPANY
301 Henrietta Street
Kalamazoo, Michigan 49001(US)

(72) Inventor: McCall, John M.
c/o The Upjohn Company 301 Henrietta Street
Kalamazoo Michigan 49001(US)
Inventor: Luderer, John R.
c/o The Upjohn Company 301 Henrietta Street
Kalamazoo Michigan 49001(US)
Inventor: Kane, Paula F.
c/o The Upjohn Company 301 Henrietta Street
Kalamazoo Michigan 49001(US)
Inventor: Gibson, J. Kenneth
c/o The Upjohn Company 301 Henrietta Street
Kalamazoo Michigan 49001(US)

(74) Representative: Perry, Robert Edward et al
GILL JENNINGS & EVERY 53-64 Chancery Lane
London WC2A 1HN(GB)

## (54) Antiarrhythmic use of aminocycloalkylamides.

(57) Benzamides, benzeneacetamides, and spirocyclobenzamides and -benzeneacetamides of the formula

R3, R4 – C[(CH2)p, (CH2)n] – cyclohexane ring bearing N(R)-C(=E)-A-Q and N(R1)(R2)   (I)

which were previously disclosed as having analgesic utility been found to have antiarrhythmic utility.



Xerox Copy Centre

# ANTIARRHYTHMIC USE OF AMINOCYCLOALKYLAMIDES

## BACKGROUND OF THE INVENTION

Cardiac arrhythmias both atrial and ventricular have been known and treated for years. Arrhythmias require therapy because they produce a variety of unpleasant and/or harmful symptoms, can lead to sudden cardiac death and contribute to unfavorable hemodynamics. In many cases individuals may have arrhythmias from birth and live an almost normal life without any serious cardiac problem. On the other hand, certain arrhythmias have been associated with and are believed to be associated with the sudden cardiac death syndrome in which an individual suddenly experiences a disturbance of cardiac rhythm and death. The ventricular arrhythmia is all the more serious or risky in an individual who has an existing heart disease or previous heart attack.

Numerous antiarrhythmic drugs are available for treating arrhythmias. They have even been classified into four groups or classes based on the dominant electrophysiological property of each drug. The most common of the antiarrhythmic drugs include quinidine, procainamide (The Merck Index, Tenth Edition, Merck & Co., Inc., Rahway, NJ, 1983, Monograph number 7658), disopyramide (number 3378), phenytoin (number 7204), lidocaine (number 5310), mexiletine (number 6047), encainide (US Patent 3,931,195), tocainide (number 9319), lorcainide (number 5401), N-acetylprocainamide (number 14), ethmozin (number 6121), propranolol (number 7740), metoprolol (number 6027), nadolol (number 6195), timolol, atenolol (number 868), bretylium (number 1348), amiodarone (number 491), sotolol (number 8571) flecainide (number 4019) and indecainide and verapamil (number 9747).

US Patents 4,098,904, 4,145,435 and 4,438,130 claimed benzamide, benzeneacetamide and spirocyclobenzamide and -benzeneacetamide type compounds, respectively, which were disclosed as having analgesic utility. These compounds as well as other known amides disclosed in US Patents 4,360,531, 4,359,476, 4,466,977, 4,460,600 and in European Patent Applications 84303277.2 and 84303698.9 and US patent application Serial No. 614,408 filed May 25, 1984 have surprisingly and unexpectedly been found to be useful as antiarrhythmic agents.

It is known that naloxone, a agonist/antagonist analgesic has antiarrhythmic properties. This was presented by J. W. Holiday of Walter Reed Army Hospital Medical Center at a Neuroscience Meeting in October 1984.

Many of the known antiarrhythmic drugs contain aromatic, amide and amine (primary, secondary and tertiary) functionality. Substituted benzamide type antiarrhythmics include encainide hydrochloride, acecainide, capobenate sodium, capobenic acid, flecainide acetate, procainamide hydrochloride, tocainide and tolamolol.

## SUMMARY OF THE INVENTION

Disclosed is a method of treating the rhythm of a human heart in an individual who has an arrhythmia which comprises administering an antiarrhythmic effective amount of an aminocycloalkylamide of formula (I) and pharmaceutically acceptable salts thereof.

Also disclosed is a method of maintaining the normal rhythm in an individual who has had an arrhythmia which comprises administering an antiarrhythmic effective amount of an aminocycloalkylamide of formula (I) and pharmaceutically acceptable salts thereof.

Further disclosed is a method of preventing arrhythmias in an individual who is at risk of developing an arrhythmia which comprises administering an antiarrhythmic effective amount of an aminocycloalkylamide of formula (I) and pharmaceutically acceptable salts thereof.

Additionally disclosed is a method of preventing sudden cardiac death in a human where the sudden death is precipitated by a disorder of the cardiac rhythm or conduction which comprises administering an antiarrhythmic effective amount of an aminocycloalkylamide of formula (I) or pharmaceutically acceptable salts thereof.

DETAILED DESCRIPTION OF THE INVENTION

Some individuals are born with arrhythmias and others develop them sometime during their life span. There are various types of arrhythmias (atrial, ventricular, supraventricular, and junctional). The arrhythmias alone are not usually fatal to an individual. It is believed that during a ventricular arrhythmia something will cause a heart to begin to fibrillate which is the immediate cause of sudden cardiac death which claims about 400,000 victims each year in the United States. Other type of cardiac arrhythmias have also been associated with sudden cardiac death. It is further known that individuals which have or have had a cardiac disease or conditions of various types, coronary artery disease, myocardial infarction, ischemic disease, congestive heart failure, congenital heart disease, valvular heart disease, cardiomyopathy, pericardial disease, myocarditis, drug induced arrhythmias etc. and have arrhythmias have an ominous risk profile and are at a high risk of sudden cardiac death. Medical belief is that the frequency of ventricular fibrillation can be decreased by preventing, if possible, or at least decreasing the frequency of ventricular arrhythmias. The method of treatment of the present invention improves the cardiac rhythm of an individual who has arrhythmias by decreasing the frequency of arrhythmic events, thereby decreasing the likelihood of ventricular tachycardia, fibrillation, asystole and/or other rhythm disturbances which in turn prevents an adequate cardiac output, thereby reducing the risk of sudden cardiac death. The method also reduces the number and severity of arrhythmic events as well as prevents future arrhythmic events. The method of treatment of the present invention would also decrease the number, frequency and/or severity of annoying patient symptoms resulting from arrhythmias such as palpitations, fainting and syncope as well as prevents future symptoms.

The method of treatment of the present invention is also useful in maintaining the normal rhythm in an individual who previously had an arrhythmia but does not at the present time. It is particularly important to maintain the normal rhythm by preventing recurrence of an arrhythmia that had been treated initially by electrical cardioversion.

Further, the method of treatment of the present invention is useful in preventing sudden cardiac death in a human where the sudden cardiac death is precipitated by a disorder of the cardiac rhythm or conduction. Usually the disorder of cardiac rhythm is an arrhythmia; and the arrhythmia is usually a ventricular arrhythmia.

Also, the present invention is useful in preventing arrhythmias in an individual who has never had an arrhythmia but who is at risk of developing an arrhythmia. The cardiac risk usually being cardiac disease or one of the many cardiac conditions listed above.

The benzamide, benzeneacetamide and spirocyclobenzamide and-benzeneacetamide and other amides within the scope of the compounds of formula (I) are known, see US Patents 4,098,904, 4,145,435, 4,438,130, 4,360,531, 4,359,476, 4,466,977, 4,460,600 and European Patent Applications 84303277.2 and 84303698.9 and US patent application Serial No. 614,408 filed May 25, 1984. A new and better way to open the aziridine of US Patents 4,145,435 (colums 16 and 17) or 4,098,904 (columns 18 and 19) with ammonia to produce the 1,2-diamine for preparation of the aminocycloalkylamide (I) is by reaction with liquid ammonia in a sealed tube at 20-25° overnight. The excess ammonia is removed and the solid residue is recrystallized, see Example 3.

The aminocycloalkylamides (I), which are useful as antiarrhythmic agents have two asymmetric centers if $R_3$ equals $R_4$ (and three asymmetric centers if $R_3$ does not equal $R_4$) in the cycloalkyl ring which can independently have R or S configuration (Cahn-Ingold-Prelog). Given that there are two asymmetric centers, two pairs of diastereoisomers [RR, SS and RS, SR] or four isomers exist for each compound. The trans and cis pairs are readily separable. The enantiomers in each pair of diastereoisomers can be separated as is well known to those skilled in the art, see Example 1. The use of all such stereoisomers in racemic, optically pure or partially resolved form is included within the scope of this invention.

The aminocycloalkylamides (I), and combinations thereof, and pharmaceutically acceptable salts thereof are used to treat and/or prevent arrhythmias, to correct the rhythm of the human heart, to prevent sudden cardiac deaths and to treat the symptoms resulting from arrhythmias generally in the same manner and same way as other antiarrhythmic drugs are used, see US Patent 3,931,195 (encainide), Heart Disease, A Textbook of Cardiovascular Medicine, Second Edition, Vol. 1, Edited by E. Braunwald, W. B. Saunders, Philadelphia, 1984 and The Medical Letter, Vol. 25 (Issue 630) March 4, 1983.

Pharmaceutically acceptable salts include hydrochloride, hydrobromide, hydroiode, sulfate, methanesulfonate, toluenesulfonate, acetate, fumarate, phosphate, lactate, citrate, succinate, benzoate, salicylate, pamoate, cyclohexansulfamate, maleate and the alike. Preferred is the methanesulfonate salt.

These agents may be used in combination with each other and/or in combination with other known antiarrhythmic agents.

The antiarrhythmic agents (I) may be given by oral or parenteral routes, including intramuscular, intraperitoneal, subcutaneous, transtracheal, transcutaneous, and intravenous administration in appropriate dosage forms. For normal therapeutic purposes in the prevention or treatment of arrhythmias, oral or intravenous administration is preferred. Generally, treatment is commenced with a unit dose smaller than anticipated for optimum control of the arrhythmia. Thereafter, the dosage is increased by small increments until the optimal effect is reached. Smaller dosage units are generally required for intravenous treatment than are required for oral treatment. The dosage amount administered is preferably at an effective level which is without harmful or deleterious side effect on the patient.

The antiarrhythmic agents (I) are generally given in a dosage range of from about 0.1 to about 1000 mg/kg/day, preferably about 1.0 to 500 mg/kg/day, more preferably 10 to 100 mg/kg/day, administered from 1 thru 4 times a day, preferably once or twice a day, more preferably once a day.

In some instances it may be necessary or desirable to administer a larger loading dose in order to initiate therapy and then to administer smaller maintenance doses thereafter.

The exact dosage and frequency of administration depends on the particular antiarrhythmic agent (I) used, the particular condition being treated/prevented, the severity of the condition being treated/prevented, the age, weight, general physical condition of the patient, other medication the individual may be taking as is well known to those skilled in the art and can be more accurately determined by measuring the concentration of the antiarrhythmic agent (I) in the patient's blood.

## DEFINITIONS

The definitions and explanations below are for the terms as used throughout the entire patent application including both the specification and the claims.

All temperatures are in degrees Centigrade.

TLC refers to thin-layer chromatography.

Saline refers to an aqueous saturated sodium chloride solution.

Ether refers to diethyl ether.

Alcohol refers to ethyl alcohol.

Pharmaceutically acceptable refers to those properties and/or substances which are acceptable to the patient from a pharmacological/toxicological point of view and to the manufacturing pharmaceutical chemist from a physical-chemical point of view regarding composition, formulation, stability, patient acceptance and bioavailability.

When solvent pairs are used, the ratios of solvents used are volume/volume (v/v).

~ indicates the attached group can be in either possible orientation.

.... is a single or double bond.

When the term "alkyl of ___ thru ___ carbon atoms" is used, it means and includes isomers thereof where such exist.

A is:

(1) $-(CH_2)q-$, where q is 1, 2, 3 or 4,

(2) $-CH(CH_3)-$,

(3) nothing; E is an oxygen atom or bivalent sulfur;

E′ is an oxygen atom or bivalent sulfur;

E″ is an oxygen atom or bivalent sulfur;

G is:

(a) hydroxyl,

(b) $C_1$-$C_3$ alkyl,

(c) $C_1$-$C_3$ alkoxy,

(d) $C_1$-$C_3$ alkanoyloxy; J and J′ are:

(a) a hydrogen atom,

(b) hydroxy,

(c) $C_1$-$C_3$ alkyl,

(d) $C_1$-$C_3$ alkyloxy,

(e) $C_1$-$C_3$ alkanoyloxy, with the proviso that either J or J′ must be a hydrogen atom; M is an oxygen atom or bivalent sulfur;

Q is:

(1) 1-naphthyl,

(2) 2-naphthyl,

(3) $-C_6H_3(X)(Y)$; R is:
(1) a hydrogen atom,
(2) $C_1$-$C_3$ alkyl; $R_1$ is:
(1) a hydrogen atom,
(2) $C_1$-$C_3$ alkyl,
(3) $-C(R_a)(R_b)-C(R_c)=C(R_d)(R_e)$ where $R_a$, $R_b$, $R_c$, $R_d$, $R_e$ are the same or different and are a hydrogen atom or alkyl group with the proviso that $R_a + R_b + R_c + R_d + R_e$ is not more than 3 carbon atoms; $R_2$ is:
(1) a hydrogen atom,
(2) $C_1$-$C_{10}$ alkyl,
(3) $C_3$-$C_{12}$ cycloakyl,
(4) $C_4$-$C_8$ cycloalkyl $C_1$-$C_4$ alkylene,
(5) $CF_3CH_2$-,
(6) $-C(R_f)(R_g)-C(R_h)=C(R_i)(R_j)$ where $R_f$, $R_g$, $R_h$, $R_i$, $R_j$ are the same or different and are a hydrogen atom or alkyl group with the proviso= that $R_f + R_g + R_h + R_i + R_j$ is not more than 3 carbon atoms
(7) $-CH_2$-(hydroxyalkyl) where the alkyl portion is from 1 thru 4 carbon atoms,
(8) $-CH_2(CH_2)_aCH_2$-phenyl, where a is 0 or 1,
(9) $-CH_2-C^*H=CH-CH=CH-M^*$, (F-A) where the * atoms are bonded to each other to form a ring, where M is an oxygen atom or bivalent sulfur; the group $-NR_1R_2$ is selected from the group consisting of:
(1) $-N^*-(CH_2)_b-CH-(G)-C^*H_2$, (F-B), where the * atoms are bonded to each other to form a ring, where b is 1 or 2 and where G is:
(a) hydroxyl,
(b) $C_1$-$C_3$ alkyl,
(c) $C_1$-$C_3$ alkoxy,
(d) $C_1$-$C_3$ alkanoyloxy;
(2) $-N^*-CH_2-CH_2-CH(J')-CH(J)-C^*H_2$, (F-C), where the * atoms are bonded to each other to form a ring, where J and J′ are:
(a) a hydrogen atom,
(b) hydroxy,
(c) $C_1$-$C_3$ alkyl,
(d) $C_1$-$C_3$ alkyloxy,
(e) $C_1$-$C_3$ alkanoyloxy, with the proviso that either J or J′ must be a hydrogen atom;
(3) $-N^*-(CH_2)_y-N(R_{10})-(CH_2)_x-C^*H$, (F-D), where the * atoms are bonded to each other to form a ring, where x is 0 or 1, where y is 2, 3 or 4 with the proviso that x and y together are 2, 3 or 4, where $R_1$ is a hydrogen atom or $C_1$-$C_3$ alkyl,
(4) pyrrolyl,
(5) 3-pyrrolin-1-yl,
(6) 3-azabicyclo[3.1.0]hexan-3-yl,
(7) 3-azabicyclo[3.2.0]heptan-3-yl; $R_3$ is:
(1) a hydrogen atom,
(2) a hydroxyl group,
(3) $C_1$-$C_3$ alkyloxy,
(4) $R_9$-CO-O-, where $R_9$ is a hydrogen atom or $C_1$-$C_3$ alkyl,
(5) mercapto,
(6) $C_1$-$C_3$ alkylthio; $R_4$ is:
(1) a hydrogen atom,
(2) $C_1$-$C_3$ alkyloxy,
(3) $C_1$-$C_3$ alkylthio; or $R_3$ and $R_4$ taken together are:
(1) $-Z-(CH_2)m-$, where Z is an oxygen atom, bivalent sulfur or sulfinyl group and where m is 3 or 4,
(2) $-CH_2-Z-(CH_2)r-$, where r is 2 or 3,
(3) $=E'$, where E′ is an oxygen atom or bivalent sulfur,
(4) $-E'-(C_2$-$C_5$ alkyl$)-_{E''}-$, where E″ is an oxygen atom or bivalent sulfur,
(5) $=N \sim OH$,

(6) =(N~O-CO-$CH_3$, with the proviso that when $R_3$ is a hydrogen atom $R_4$ may form part of a carbon-carbon double bond with an adjacent carbon atom which does not bear the -$NR_1R_2$ or -NR moiety of formula (I);

$R_5$ is a hydrogen atom or $C_1$-$C_2$ alkyl;

$R_6$ and $R_7$ are the same or different and are:

(i) a hydrogen atom or

(ii) $C_1$-$C_3$ alkyl; $R_8$ is

(i) a hydrogen atom or

(ii) $C_1$-$C_2$ alkyl; $R_9$ is a hydrogen atom or $C_1$-$C_3$ alkyl;

$R_{10}$ is a hydrogen atom or $C_1$-$C_3$ alkyl;

$R_a$, $R_b$, $R_c$, $R_d$, $R_e$ are the same or different and are a hydrogen atom or alkyl group with the proviso that $R_a + R_b + R_c + R_d + R_e$ is not more than 3 carbon atoms;

$R_f$, $R_g$, $R_h$, $R_i$, $R_j$ are the same or different and are a hydrogen atom or alkyl group with the proviso that $R_f + R_g + R_h + R_i + R_j$ is not more than 3 carbon atoms;

$R_k$, $R_l$, $R_m$, $R_n$, $R_o$ are the same or different and are a hydrogen atom or alkyl group with the proviso that $R_k + R_l + R_m + R_n + R_o$ is not more than 3 carbon atoms;

X and Y are the same or different and are

(a) a hydrogen atom,

(b) a fluorine, chlorine or bromine atom,

(c) cyano,

(d) sulfonic acid,

(e) methanesulfonyl,

(f) $R_5$-CO-, where $R_5$ is a hydrogen atom or $C_1$-$C_2$ alkyl,

(g) hydroxy,

(h) trifluoromethyl,

(i) $C_1$-$C_3$ alkyl,

(j) $C_1$-$C_3$ alkoxy,

(k) phenyl,

(l) -$NR_6R_7$, where $R_6$ and $R_7$ are the same or different and are:

(i) a hydrogen atom or

(ii) $C_1$-$C_3$ alkyl,

(m) nitro,

(n) azido,

(o) $C_1$-$C_3$ alkoxycarbonyl,

(p) $C_1$-$C_3$ alkanoyloxy,

(q) -NH-CO-$R_8$, where $R_8$ is

(i) a hydrogen atom or

(ii) $C_1$-$C_2$ alkyl,

(r) benzoyl,

(s) HCOO-,

(t)-C($R_k$)($R_l$)-C($R_m$)=C($R_n$)($R_o$) where $R_k$, $R_l$, $R_m$, $R_n$, $R_o$ are the same or different and are a hydrogen atom or alkyl group with the proviso that $R_k + R_l + R_m + R_n + R_o$ is not more than 3 carbon atoms. Z is:

(1) an oxygen atom,

(2) bivalent sulfur,

(3) sulfinyl; a is 0 or 1;

b is 1 or 2;

m is 3 or 4;

n is 0 thru 9;

p is 0 thru 9;

q is 1, 2, 3 or 4;

r is 2 or 3;

x is 0 or 1;

y is 2, 3 or 4;

(A) with the proviso that when one or both of $R_3$ and $R_4$ is not hydrogen p is 0 thru 4, n is 0 thru 4 such that the resulting cycloaliphatic ring has 5, 6 or 7 carbon atoms;

(B) with the proviso that when $R_3$ and $R_4$ are both hydrogen, the resulting cycloaliphatic ring has 5 thru 12 carbon atoms;

(C) with the proviso that when p is 2, n is 1, m is 3, A is $(-CH_2)_q$-where q is 1, R is methyl, $R_1$ and $R_2$ are taken together with the nitrogen to which they are bonded to complete a pyrrolidinyl ring, E is oxygen, Z is oxygen, and the relative stereochemistry is ($5\alpha$, $7\alpha$, $8\beta$), then X and Y taken together on the phenyl ring cannot be chlorine on the 2-and 4-positions of the phenyl ring.

When the term "$C_x$-$C_y$ alkyl" is used, it means an alkyl group of x thru y carbon atoms and includes isomers thereof where such exist.

When the term "$C_x$-$C_y$ cycloalkyl" is used, it means a cycloalkyl group of x thru y carbon atoms.

When the term "$C_x$-$C_y$ alkoxy" or "$C_x$-$C_y$ alkylthio" is used, it means an alkoxy or alkylthio group respectively where the alkyl portion is x thru y carbon atoms.

When the term "$C_x$-$C_y$ alkanoyloxy" is used, it means an alkanoyloxy group where the alkyl portion is x thru y carbon atoms.

When the term "$C_x$-$C_y$ alkoxycarbonyl" is used, it means an alkoxy group where the alkyl portion is x thru y carbon atoms attached to a carbonyl group.

$\phi$ refers to phenyl ($C_6H_5$).

Arrhythmia means a heart beat without any rhythm. It can also mean a single, abnormal heart beat, or groups of abnormal heart beats.

Dysrhythmia means a rhythmic heart beat with an incorrect rhythm.

For purposes of this patent application when the term arrhythmia or any derivative thereof is used, it means and includes both arrhythmia and dysrhythmia as defined above, or any disorder of the normal heart rhythm or disorder of normal cardiac conduction.

Treatment generally means improving, helping or preventing from getting worse an existing undesirable condition more specifically reducing the frequency, number, severity of the arrhythmia, or decreasing the likelihood that the arrhythmia will progress to another and perhaps more dangerous arrhythmia, or decreasing the likelihood that the arrhythmia will stimulate the appearance of another arrhythmia, or reducing the frequency, number or severity of unpleasant symptoms which the patient may experience as a result of the cardiac rhythm, or decreasing the likelihood that the patient will experience sudden cardiac death.

Prevention generally means stopping or decreasing the likelihood of a cardiac arrhythmia, symptoms related to that arrhythmia, or sudden death in association with arrhythmia in a patient who is judged to be at increased risk for these events.

Progression of an arrhythmia means that the arrhythmia increases in frequency or severity, or that the arrhythmia predisposes to the development of another type of arrhythmia, or that the arrhythmia converts to another type of arrhythmia.

## EXAMPLES

Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

### Example 1 Resolution of Racemic or trans-(±)-p-bromo-N-[2-(dimethylamino)cyclohexyl]benzamide

A solution of trans-(±)-p-bromo-N-[2-(dimethylamino)cyclohexyl]benzamide (I, US Patent 4,098,904, 13.6 g) is dissolved in a minimum volume of acetonitrile and is added to (-)dibenzoyl-L-tartaric acid (15.73 g) which is dissolved in a minimum amount of warm acetonitrile. The crystals are filtered and then recrystallized from hot acetonitrile to give pure diastereomeric salt. This salt is converted to the free base by partition between ether and aqueous sodium hydroxide. The organic phase is washed with saline, filtered thru sodium sulfate and concentrated. The rotation of the crystalline product is determined in methanol, $[\alpha]_D^{25}$ = +40.6°. A sample of the product is crystallized from ether to give trans-(+)-p-bromo-N-[2-(dimethylamino)-cyclohexyl]benzamide, m.p. 148-149°.

Example 2 Trans-(±)-p-bromo-N-[2-(amino)cyclohexyl]benzamide (I)

7-Azabicyclo[4.1.0]heptane (15 g) is stirred with triethylamine (17.3 g) in dry ether (450 ml). An ether solution of 4-bromobenzoyl chloride is added at 20-25° over 2.5 hr. The reaction mixture is stirred for 2 hr more and then partitioned with aqueous sodium bicarbonate. The organic phase is washed with saline, dried over sodium sulfate and concentrated to give crude 7-(p-bromobenzoyl)-7-azabicyclo[4.1.0]heptane. Dioxane (100 ml) and concentrated ammonium hydroxide (120 ml) are added and the mixture is stirred on a steam bath for 20 hr. The reaction is extracted with chloroform (500 ml). The organic phase is washed with aqueous sodium bicarbonate, dried over sodium sulfate and concentrated. TLC, methanol/methylene chloride (8/92 with a drop of ammonium hydroxide) shows the product has formed. The residue is crystallized from hot ethyl acetate, and recrystallized again to give the title compound, mp 170-171°.

Example 3 Trans-(±)-p-bromo-N-[2-(amino)cyclohexyl]benzamide (I)

7-(p-Bromobenzoyl)-7-azabicyclo[4.1.0]heptane (30 g) is combined in portions with liquid ammonia (500 ml) in a pressure tube at -78°, the tube is sealed and the mixture stirred at 20-25° for 19 hr. The tube is cooled to -78°, opened, and the liquid ammonia allowed to evaporate under a stream of nitrogen at 20-25° overnight. The solid is transferred from the tube in ethyl acetate (700 ml) and the mixture warmed to give a solution which is filtered. The filtrate is cooled, the solid recovered and recrystallized from ethyl acetate to give the title compound, mp 169.5-171°.

Example 4 Trans-(±)-p-bromo-N-[2-(amino)cyclohexyl]benzamide methanesulfonate (I)

Trans-(±)-p-bromo-N-[2-(amino)cyclohexyl]benzamide (I, Example 3, 38 g) in methanol (450 ml) is treated with methane sulfonic acid (8.3 ml). The mixture is warmed on a steam bath to give a solution which is filtered thru cotton. The cotton is washed with warm methanol (150 ml) The filtrate is placedon a steam bath and treated with ethyl acetate in portions over a few minutes to a volume of 1600 ml. A precipitate begins to form and on continued heating the precipitate thickens. The mixture is permitted to sit at 20-25° then cooled in an ice bath and filtered. The filtrate is concentrated to about 400 ml on the rotary evaporator, giving a residue which is warmed on the steam bath to give a solution. The solution is treated with ethyl acetate (200 ml) to give a solid. This solid and the previously obtained solid are combined and recrystallized to give the title compound, mp 279-281 (soften 276).

Example 5 Trans p-bromo-N-[2-(dimethylamino)cyclohexyl]benzamide maleate (I)

Following the general procedure of Example 4 and making noncritical variations but using trans p-bromo-N-[2-(dimethylamino)cyclohexyl]benzamide (I, US Patent 4,098,904) and maleic acid, the title compound is obtained.

Example 6 Trans-(+)-p-bromo-N-[2-(amino)cyclohexyl]benzamide (I) and trans-(-)-p-bromo-N-[2-(amino)-cyclohexyl]benzamide (I)

Following the general procedure of US Patent 4,098,904 and Example 1 above and making noncritical variations but starting with (racemic) trans-(±)-p-bromo-N-[2-(amino)cyclohexyl]benzamide (I) the title compounds are obtained.

Example 7 Trans-(+)-p-bromo-N-[2-(amino)cyclohexyl]benzamide methanesulfonate (I) and trans-(-)-p-bromo-N-[2-(amino) cyclohexyl]benzamide methanesulfonate (I)

Following the general procedure of Example 4 and making noncritical variations but starting with trans-(+)-p-bromo-N-[2-(amino)cyclohexyl]benzamide (I, Example 6) and trans-(-)-p-bromo-N-[2-(amino)-cyclohexyl]benzamide (I, Example 6) the title compounds are obtained.

ENUMERATED EMBODIMENTS

1. A method of maintaining the normal rhythm in an individual who has has an arrhythmia which comprises administering an antiarrhythmic effective amount of an aminocycloalkylamide of the formula

(I)

and pharmaceutically acceptable salts thereof where A, E, E', E", G, J, J', M, Q, R, $R_1$, $R_2$, $R_3$, $R_4$, $R_5$, $R_6$, $R_7$, $R_8$, $R_9$, $R_{10}$, $R_a$, $R_b$, $R_c$, $R_d$, $R_e$, $R_f$, $R_g$, $R_h$, $R_i$, $R_j$, $R_k$, $R_l$, $R_m$, $R_n$, $R_o$, X, Y, Z, a, b, m, n, p, q, r, x, y and ~ are defined in the specification.

2. A method according to Enumerated Embodiment 1 where the method of maintaining the normal ryhthm is preventing the recurrence of an arrhythmia that had been treated initially by electrical cardioversion.

3. A method according to Enumerated Embodiment 1 where the aminocycloalkylamide (I) is selected from the group consisting of trans-(±)-p-bromo-N-[2-(dimethylamino)cyclohexyl]benzamide, trans-(±)-p-bromo-N-[2-(amino)cyclohexyl]-benzamide, trans-(+)-p-bromo-N-[2-(dimethylamino)cyclohexyl]benzamide, trans-(+)-p-bromo-N-[2-(amino)cyclohexyl]benzamide, trans-(-)-p-bromo-N-[2-(amino)cyclohexyl]benzamide and pharmaceutically acceptable salts thereof.

4. A method according to Enumerated Embodiment 1 where the aminocycloalkylamide (I) is trans-(±)-p-bromo-N-[2-(amino)cyclohexyl]benzamide, trans-(+)-p-bromo-N-[2-(amino)cyclohexyl]benzamide and trans-(-)-p-bromo-N-[2-(amino)cyclohexyl]benzamide and pharmaceutically acceptable salts thereof.

5. A method according to Enumerated Embodiment 1 where the aminocycloalkylamide (I) is trans-(±)-p-bromo-N[2-(amino)cyclohexyl]benzamide maleate, trans-(+)-p-bromo-N-[2-(amino)cyclohexyl]benzamide maleate, trans-(-)-p-bromo-N-[2-(amino)cyclohexyl]benzamide maleate, trans-(±)-p-bromo-N-[2-(amino)-cyclohexyl]benzamide methanesulfonate, trans-(+)-p-bromo-N-[2-(amino)cyclohexyl]benzamide methanesulfonate and trans-(-)-p-bromo-N-[2-(amino)cyclohexyl]benzamide methanesulfonate.

6. A method according to Claim 1 where the pharmaceutically acceptable salt is selected from the group consisting of hydrochloride, hydrobromide, hydroiode, sulfate, methanesulfonate, tolunesulfonate, acetate, fumarate, phosphate, lactate, citrate, succinate, benzoate, salicylate, pamoate, cyclohexansulfamate and maleate.

7. A method according to Enumerated Embodiment 1 where the aminocycloalkylamide (I) is administered once or twice a day.

8. A method according to Enumerated Embodiment 1 where the antiarrhythmic effective amount is from about 0.1 to about 1000 mg/kg/day.

9. A method according to Enumerated Embodiment 1 where the antiarrhythmic effective amount is from about 1.0 to about 500 mg/kg/day.

10. A method according to Enumerated Embodiment 1 where the antiarrhythmic effective amount is from about 10 to 100 mg/kg/day.

11. A method according to Enumerated Embodiment 1 where the aminocycloalkylamide (I) is given orally or intravenously.

12. A method of preventing arrhythmias in an individual who is at risk of developing an arrhythmia which comprises administering an antiarrhythmic effective amount of an aminocycloalkylamide of the formula

$$\text{(I)}$$

and pharmaceutically acceptable salts thereof where A, E, E', E", G, J, J', M, Q, R, $R_1$, $R_2$, $R_3$, $R_4$, $R_5$, $R_6$, $R_7$, $R_8$, $R_9$, $R_{10}$, $R_a$, $R_b$, $R_c$, $R_d$, $R_e$, $R_f$, $R_g$, $R_h$, $R_i$, $R_j$, $R_k$, $R_l$, $R_m$, $R_n$, $R_o$, X, Y, Z, a, b, m, n, p, q, r, x, y and ~ are defined in the specification.

13. A method according to Enumerated Embodiment 12 where the arrhythmia is a ventricular arrhythmia.

14. A method according to Enumerated Embodiment 12 where the individual who is at risk has a cardiac disease or condition selected from the group consisting of coronary artery disease, myocardial infarction, ischemic disease, congestive heart failure, congenital heart disease, valvular heart disease, cardiomyopathy, pericardial disease, myocarditis, or drug induced arrhythmias.

15. A method according to Enumerated Embodiment 12 where the aminocycloalkylamide (I) is selected from the group consisting of trans-(±)-p-bromo-N-[2-(dimethylamino)cyclohexyl]benzamide, trans-(±)-p-bromo-N-[2-(amino)cyclohexyl]-benzamide, trans-(+)-p-bromo-N-[2-(dimethylamino)cyclohexyl]-benzamide, trans-(+)-p-bromo-N-[2-(amino)cyclohexyl]benzamide, trans-(-)-p-bromo-N-[2-(amino)-cyclohexyl]benzamide and pharmaceutically acceptable salts thereof.

16. A method according to Enumerated Embodiment 12 where the aminocycloalkylamide (I) is trans-(±)-p-bromo-N-[2-(amino)cyclohexyl]benzamide, trans-(+)-p-bromo-N-[2-(amino)cyclohexyl]benzamide and trans-(-)-p-bromo-N-[2-(amino)cyclohexyl]benzamide and pharmaceutically acceptable salts thereof.

17. A method according to Enumerated Embodiment 12 where the aminocycloalkylamide (I) is trans-(±)-p-bromo-N-[2-(amino)cyclohexyl]benzamide maleate, trans-(+)-p-bromo-N-[2-(amino)cyclohexyl]-benzamide maleate, trans-(-)-p-bromo-N-[2-(amino)cyclohexyl]benzamide maleate, trans-(±)-p-bromo-N-[2-(amino)cyclohexyl]benzamide methanesulfonate, trans-(+)-p-bromo-N-[2-(amino)cyclohexyl]benzamide methanesulfonate and trans-(-)-p-bromo-N-[2-(amino)cyclohexyl]benzamide methanesulfonate.

18. A method according to Claim 1 where the pharmaceutically acceptable salt is selected from the group consisting of hydrochloride, hydrobromide, hydroiode, sulfate, methanesulfonate, tolunesulfonate, acetate, fumarate, phosphate, lactate, citrate, succinate, benzoate, salicylate, pamoate, cyclohexansulfamate and maleate.

19. A method according to Enumerated Embodiment 12 where the aminocycloalkylamide (I) is administered once or twice a day.

20. A method according to Enumerated Embodiment 12 where the antiarrhythmic effective amount is from about 0.1 to about 1000 mg/kg/day.

21. A method according to Enumerated Embodiment 12 where the antiarrhythmic effective amount is from about 1.0 to about 500 mg/kg/day.

22. A method according to Enumerated Embodiment 12 where the antiarrhythmic effective amount is from about 10 to 100 mg/kg/day.

23. A method according to Enumerated Embodiment 12 where the aminocycloalkylamide (I) is given orally or intravenously.

24. A method of preventing sudden cardiac death in a human where the sudden death is precipitated by a disorder of the cardiac rhythm or conduction which comprises administering an antiarrhythmic effective amount of an aminocycloalkylamide of the formula

(I)

and pharmaceutically acceptable salts thereof where A, E, E′, E″, G, J, J′, M, Q, R, $R_1$, $R_2$, $R_3$, $R_4$, $R_5$, $R_6$, $R_7$, $R_8$, $R_9$, $R_{10}$, $R_a$ , $R_b$, $R_c$, $R_d$, $R_e$ , $R_f$, $R_g$, $R_h$, $R_i$ , $R_j$, $R_k$, $R_l$, $R_m$ , $R_n$, $R_o$, X, Y, Z, a, b, m, n, p, q, r, x, y and ~ are defined in the specification.

25. A method according to Enumerated Embodiment 24 where the disorder of the cardiac rhythm is an arrhythmia.

26. A method according to Enumerated Embodiment 25 where the arrhythmia is a ventricular arrhythmia.

27. A method according to Enumerated Embodiment 24 where the aminocycloalkylamide (I) is selected from the group consisting of trans-(±)-p-bromo-N-[2-(dimethylamino)cyclohexyl]benzamide, trans-(±)-p-bromo-N-[2-(amino)cyclohexyl]-benzamide, trans-(+)-p-bromo-N-[2-(dimethylamino)cyclohexyl]-benzamide, trans-(+)-p-bromo-N-[2-(amino)cyclohexyl]benzamide, trans-(-)-p-bromo-N-[2-(amino)-cyclohexyl]benzamide and pharmaceutically acceptable salts thereof.

28. A method according to Enumerated Embodiment 24 where the aminocycloalkylamide (I) is trans-(±)-p-bromo-N-[2-(amino)cyclohexyl]benzamide, trans-(+)-p-bromo-N-[2-(amino)cyclohexyl]benzamide and trans-(-)-p-bromo-N-[2-(amino)cyclohexyl]benzamide and pharmaceutically acceptable salts thereof.

29. A method according to Enumerated Embodiment 24 where the aminocycloalkylamide (I) is trans-(±)-p-bromo-N-[2-(amino)cyclohexyl]benzamide maleate, trans-(+)-p-bromo-N-[2-(amino)cyclohexyl]-benzamide maleate, trans-(-)-p-bromo-N-[2-(amino)cyclohexyl]benzamide maleate, trans-(±)-p-bromo-N-[2-(amino)cyclohexyl]benzamide methanesulfonate, trans-(+)-p-bromo-N-[2-(amino)cyclohexyl]benzamide methanesulfonate and trans-(-)-p-bromo-N-[2-(amino)cyclohexyl]benzamide methanesulfonate.

30. A method according to Enumerate Embodiment 24 where the pharmaceutically acceptable salt is selected from the group consisting of hydrochloride, hydrobromide, hydroiode, sulfate, methanesulfonate, tolunesulfonate, acetate, fumarate, phosphate, lactate, citrate, succinate, benzoate, salicylate, pamoate, cyclohexansulfamate and maleate.

31. A method according to Enumerated Embodiment 24 where the aminocycloalkylamide (I) is administered once or twice a day.

32. A method according to Enumerated Embodiment 24 where the antiarrhythmic effective amount is from about 0.1 to about 1000 mg/kg/day.

33. A method according to Enumerated Embodiment 24 where the antiarrhythmic effective amount is from 1.0 to about 500 mg/kg/day.

34. A method according to Enumerated Embodiment 24 where the antiarrhythmic effective amount is from about 10 to 100 mg/kg/day.

35. A method according to Enumerated Embodiment 24 where the aminocycloalkylamide (I) is given orally or intravenously.

36. A method according to Enumerated Embodiment 1 where the aminocycloalkylamide (I) is trans-(±)-p-bromo-N-[2-(amino)cyclohexyl]benzamide methanesulfonate.

37. A method according to Enumerated Embodiment 12 where the aminocycloalkylamide (I) is trans-(±)-p-bromo-N-[2-(amino)cyclohexyl]benzamide methanesulfonate.

38. A method according to Enumerated Embodiment 24 where the aminocycloalkylamide (I) is trans-(±)-p-bromo-N-[2-(amino)cyclohexyl]benz amide methanesulfonate.

## CHART A

| Linear Formula | Chemical Structure | |
|---|---|---|
| **Formula** | | |
| $-CH_2-C^*H=CH-CH=CH-M^*$, where the * atoms are bonded to each other to form a ring | —CH2— ring with M | (F-A) |
| $-N^*-(CH_2)_b-CH(G)-C^*H_2$, where the * atoms are bonded to each other to form a ring | N, (CH2)b, CH2-G, CH2 ring | (F-B) |
| $-N^*-CH_2-CH_2-CH(J')-CH(J)-C^*H_2$, where the * atoms are bonded to each other to form a ring | N ring with J', J | (F-C) |
| $-N^*-(CH_2)_y-N(R_{10})-(CH_2)_{x-1}-C^*H_2$, where the * atoms are bonded to each other to form a ring | N, x(CH2), (CH2)y, N–R10 ring | (F-D) |

## Claims

1. Use of an aminocycloalkylamide for the manufacture of a medicament for use in treating or preventing arrhythmia or a conduction disorder, or improving or maintaining rhythm or conduction, of the human heart, in which the aminocycloalkylamide is a compound of the formula

(I)

where

A is:

(1) $-(CH_2)q-$, where q is 1, 2, 3 or 4,

(2) $-CH(CH_3)-$,

(3) nothing;

E is an oxygen atom or bivalent sulfur;

Q is:

(1) 1-naphthyl,

(2) 2-naphthyl,

(3) $-C_6H_3(X)(Y)$, where X and Y are the same or different and are

(a) a hydrogen atom,

(b) a fluorine, chlorine or bromine atom,

(c) a cyano group,

(d) a sulfonic acid group,

(e) a methanesulfonyl group,

(f) $R_5$-CO-, where $R_5$ is a hydrogen atom or $C_1$-$C_2$ alkyl,

(g) a hydroxy group,

(h) a trifluoromethyl group,

(i) $C_1$-$C_3$ alkyl,

(j) $C_1$-$C_3$ alkoxy, (k) a phenyl group,

(l) $-NR_6R_7$, where $R_6$ and $R_7$ are the same or different and are:

(i) a hydrogen atom or

(ii) $C_1$-$C_3$ alkyl,

(m) a nitro group,

(n) a azido group,

(o) $C^1$-$C_3$ alkoxycarbonyl,

(p) $C^1$-$C_3$ alkanoyloxy,

(q) -NH-CO-$R_8$, where $R_8$ is

(i) a hydrogen atom or

(ii) $C_1$-$C_2$alkyl,

(r) a benzoyl group,

(s) HCOO-,

(t)-$C(R_k)(R_1)$-$C(R_m)=C(R_n)(R_o)$ where $R_k$, $R_l$, $R_m$, $R_n$, $R_o$are the same or different and are a hydrogen atom or alkyl group with the proviso that $R_k + R_l + R_m + R_n + R_o$ is not more than 3 carbon atoms

R is:

(1) a hydrogen atom,

(2) $C_1$-$C_3$ alkyl,

$R_1$ is:
(1) a hydrogen atom,
(2) $C_1$-$C_3$ alkyl,
(3) -$C(R_a)(R_b)$-$C(R_c)=C(R_d)(R_e)$ where $R_a$, $R_b$, $R_c$, $R_d$ , $R_e$ are the same or different and are a hydrogen atom or alkyl group with the proviso that $R_a + R_b + R_c + R_d + R_e$ is not more than 3 carbon atoms;
$R_2$ is:
(1) a hydrogen atom,
(2) $C_1$-$C_{10}$ alkyl,
(3) $C_3$-$C_{12}$ cycloalkyl,
(4) $C_4$-$C_8$ cycloalkyl $C_1$-$C_4$ alkylene,
(5) $CF_3CH_2$-,
(6) -$C(R_f)(R_g)$-$C(R_h)=C(R_i)(R_j)$ where $R_f$, $R_g$, $R_h$, $R_i$, $R_j$ are the same or different and are a hydrogen atom or alkyl group with the proviso that $R_f + R_g + R_h + R_i + R_j$ is not more than 3 carbon atoms
(7) -$CH_2$-(hydroxyalkyl) where the alkyl portion is from 1 thru 4 carbon atoms,
(8) -$CH_2(CH_2)_aCH_2$-phenyl, where a is 0 or 1,
(9) -$CH_2$-$C^*H=CH$-$CH=CH$-$M^*$, (F-A) where the * atoms are bonded to each other to form a ring, where M is an oxygen atom or bivalent sulfur;
the group -$NR_1R_2$ is selected from the group consisting of:
(1) -$N^*$-$(CH_2)_b$-CH-(G)-$C^*H_2$, (F-B), where the * atoms are bonded to each other to form a ring, where b is 1 or 2 and
where G is:
(a) hydroxyl,
(b) $C_1$-$C_3$ alkyl,
(c) $C_1$-$C_3$ alkoxy,
(d) $C_1$-$C_3$ alkanoyloxy;
(2) -$N^*$-$CH_2$-$CH_2$-CH(J')-CH(J)-$C^*H_2$, (F-C), where the * atoms are bonded to each other to form a ring, where J and J' are:
(a) a hydrogen atom,
(b) hydroxy,
(c) $C_1$-$C_3$ alkyl,
(d) $C_1$-$C_3$ alkyloxy,
(e) $C_1$-$C_3$ alkanoyloxy, with the proviso that either J or J' must be a hydrogen atom;
(3) -$N^*$-$(CH_2)_y$-$N(R_{10})$-$(CH_2)_x$-$C^*H$, (F-D), where the * atoms are bonded to each other to form a ring, where x is 0 or 1, where y is 2, 3 or 4 with the proviso that x and y together are 2, 3 or 4, where $R_{10}$ is a hydrogen atom or $C_1$-$C_3$ alkyl,
(4) pyrrolyl,
(5) 3-pyrrolin-1-yl,
(6) 3-azabicyclo[3.1.0]hexan-3-yl,
(7) 3-azabicyclo[3.2.0]heptan-3-yl;
$R_3$ is:
(1) a hydrogen atom,
(2) a hydroxyl group,
(3) $C_1$-$C_3$ alkyloxy,
(4) $R_9$-CO-O-, where $R_9$ is a hydrogen atom or $C_1$-$C_3$ alkyl,
(5) mercapto,
(6) $C_1$-$C_3$ alkylthio;
$R_4$ is:
(1) a hydrogen atom,
(2) $C_1$-$C_3$ alkyloxy,
(3) $C_1$-$C_3$ alkylthio; or
$R_3$ and $R_4$ taken together are:
(1) -Z-$(CH_2)$m-, where Z is an oxygen atom, bivalent sulfur or sulfinyl group and where m is 3 or 4,
(2) -$CH_2$-Z-$(CH_2)$r-, where r is 2 or 3,
(3) =E', where E' is an oxygen atom or bivalent sulfur,
(4) -E'-($C_2$-$C_5$ alkyl)-$_{E''}$-, where E" is an oxygen atom or bivalent sulfur,
(5) =N~OH,
(6) =(N~O-CO-$CH_3$,
with the proviso that when $R_3$ is a hydrogen atom $R_4$ may form part of a carbon-carbon double bond with an

adjacent carbon atom which does not bear the $-NR_1R_2$ or -NR moiety of formula (I);
a is 0 or 1;
n is 0 thru 9;
p is 0 thru 9;
~ indicates the attached group can be in either possible orientation;
(A) with the proviso that when one or both of $R_3$ and $R_4$ is not hydrogen p is 0 thru 4, n is 0 thru 4 such that the resulting cycloaliphatic ring has 5, 6 or 7 carbon atoms;
(B) with the proviso that when $R_3$ and $R_4$ are both hydrogen, the resulting cycloaliphatic ring has 5 thru 12 carbon atoms;
(C) with the proviso that when p is 2, n is 1, m is 3, A is $(-CH_2)_q$-where q is 1, R is methyl, $R_1$ and $R_2$ are taken together with the nitrogen to which they are bonded to complete a pyrrolidinyl ring, E is oxygen, Z is oxygen, and the relative stereochemistry is ($5\alpha$, $7\alpha$, $8\beta$), then X and Y taken together on the phenyl ring cannot be chlorine on the 2-and 4-positions of the phenyl ring;
or a pharmaceutically acceptable salt thereof.

2. Use according to claim 1, wherein the aminocycloalkylamide (I) is trans-(± or +)-p-bromo-N-[2-(dimethylamino)cyclohexyl]benzamide or trans-(±, + or -)-p-bromo-N-[2-(amino)cyclohexyl]benzamide, or a pharmaceutically acceptable salt thereof.

3. Use according to claim 1, wherein the aminocycloalkylamide (I) is trans-(±, + or -)-p-bromo-N-[2-(amino)cyclohexyl]benzamide or a pharmaceutically acceptable salt thereof.

4. Use according to any preceding claim, wherein the compound is a pharmaceutically acceptable salt which is a hydrochloride, hydrobromide, hydroiode, sulfate, methanesulfonate, toluenesulfonate, acetate, fumarate, phosphate, lactate, citrate, succinate, benzoate, salicylate, pamoate, cyclohexanesulfamate or maleate.

5. Use according to claim 4, wherein the aminocycloalkylamide (I) is trans-(±, + or -)-p-bromo-N-[2-(amino)cyclohexyl]benzamide maleate or trans-(±, + or-)-p-bromo-N-[2-(amino)cyclohexyl]benzamide methanesulfonate.

6. Use according to claim 4, wherein the aminocycloalkylamide (I) is trans (±)-p-bromo-N-[2-(amino)-cyclohexyl]benzamide methanesulfonate.

7. Use according to any preceding claim, wherein the medicament is adapted to be give orally or intravenously.

8. Use according to any preceding claim, wherein the medicament is for reducing the number, frequency or severity of arrhythmic events or for preventing arrhythmic events.

9. Use according to claim 8, wherein the medicament is for decreasing the number, frequency or severity of palpitations, faintings or syncope, or for preventing palpitations, faintings or syncope.

10. Use according to any preceding claim, wherein the arrhythmia is atrial, ventricular, supraventricular or junctional arrhythmia.

11. Use according to any preceding claim, wherein the arrhythmia is Kent bypass tract-WPW syndrome, James bypass tract-LGL syndrome or Mahaim bypass tract.